(19) Europäisches Patentamt
European Patent Office
Office européen des brevets

(11) Veröffentlichungsnummer: **0 086 389**
**B1**

# (12) EUROPÄISCHE PATENTSCHRIFT

(45) Veröffentlichungstag der Patentschrift: **10.12.86**

(51) Int. Cl.⁴: **C 07 D 487/04,** C 09 B 29/00 // (C07D487/04, 231:00, 209:00)

(21) Anmeldenummer: **83100889.1**

(22) Anmeldetag: **01.02.83**

## (54) Aminopyrazolverbindungen.

(30) Priorität: **11.02.82 DE 3204713**

(43) Veröffentlichungstag der Anmeldung: **24.08.83 Patentblatt 83/34**

(45) Bekanntmachung des Hinweises auf die Patenterteilung: **10.12.86 Patentblatt 86/50**

(84) Benannte Vertragsstaaten: **CH DE FR GB IT LI**

(56) Entgegenhaltungen:
**US - A - 3 526 633**

**Patent Abstracts of Japan Band 6, Nr. 90, 27. Mai 1982**
**JOURNAL OF THE AMERICAN CHEMICAL SOCIETY, Band 80, 20. März 1958, Easton R.H. WILEY et al. "Carbamylmaleimides from the malonamide-diethyl oxalate reaction", Seiten 1385-1388**
**Chemical Abstracts Band 91, Nr. 23, 3. Dezember 1979, Columbus, Ohio, USA W.I. AWAD et al. "Reaction of hydrazoic acid and diazo compounds with N,N'-bimaleimide and biisomaleimide", Seite 638, Spalte 2, Abstract Nr. 193223K & Egypt. J. Chem., Band 19, Nr. 2, 1976, Seiten 25**

(73) Patentinhaber: **BASF Aktiengesellschaft, Carl-Bosch-Strasse 38, D-6700 Ludwigshafen (DE)**

(72) Erfinder: **Eilingsfeld, Heinz, Dr., Pierstrasse 9a, D-6710 Frankenthal (DE)**
Erfinder: **Etzbach, Karl-Heinz, Dr., Carl-Bosch-Ring 53, D-6710 Frankenthal (DE)**

**EP 0 086 389 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Verbindungen der allgemeinen Formel I

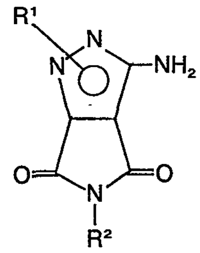

I

in der

$R^1$ Wasserstoff, $C_1$- bis $C_4$-Alkyl, Cyanethyl, Cyclohexyl, $C_1$- bis $C_4$-Alkanoyl, gegebenenfalls durch Chlor, Cyan oder Nitro substituiertes Benzoyl, $C_1$- bis $C_4$-Alkoxycarbonyl, Phenoxycarbonyl, Carbamoyl, Sulfamoyl, durch $C_1$- bis $C_4$-Alkyl oder Phenyl N-mono- oder N,N-di-sibstituiertes Carbamoyl oder Sulfamoyl, Thiocarbamoyl, $C_1$- bis $C_4$-Alkylsulfonyl, Phenyl- oder Tolylsulfonyl, gegebenenfalls durch Chlor, Cyan, Nitro, Methyl, Methoxy, Ethoxy, $C_1$- bis $C_4$-Alkanoyl, Benzoyl, $C_1$- bis $C_4$-Alkoxycarbonyl, Phenoxycarbonyl, Carboxyl, $C_1$- bis $C_4$-Alkoxysulfonyl, Phenoxysulfonyl, Carbamoyl, Sulfamoyl oder durch $C_1$- bis $C_4$-Alkyl oder Phenyl N-mono- oder N,N-disubstituiertes Carbamoyl oder Sulfamoyl substituiertes Phenyl oder Naphthyl und

$R^2$ Wasserstoff, $C_1$- bis $C_8$-Alkyl, Cyclohexyl, Allyl, $C_1$- bis $C_4$-Alkoxy-$C_2$- oder $C_3$-alkyl, Hydroxy-$C_2$- oder $C_3$-alkyl, Phenoxy-$C_2$- oder $C_3$-alkyl, Benzyl, Phenylethyl oder gegebenenfalls durch Chlor, Cyan oder Nitro substituiertes Phenyl sind.

Einzelne Reste sind neben den bereits genannten z.B. für $R^1$

Methyl, Ethyl, Propyl, Isopropyl, n-Butyl,
iso-Butyl, Phenyl, Naphthyl, o,m,p-Nitrophenyl,
o,m,p-Cyanphenyl, o,m,p-Chlorphenyl,
2,4-Dinitrophenyl, 2,4-, 3,5- und
2,6-Dichlorphenyl,
p-Methoxysulfonylphenyl, p-Ethoxysulfonylphenyl,
p-Phenoxysulfonylphenyl,
o,m,p-Ethoxycarbonylphenyl,
o,m,p-Oxycarbonylphenyl,
Acetyl, Propionyl, Butyryl, Benzoyl,
Methoxycarbonyl, Ethoxycarbonyl,
Phenoxycarbonyl, N,N-Dimethylcarbamoyl,
N-Phenylcarbamoyl, N,N-Dimethylsulfamoyl,
N-Phenylsulfamoyl, Methylsulfonyl,
Ethylsulfonyl oder p-Tolylsulfonyl

und für $R^2$

Methyl, Ethyl, Propyl, Isopropyl, Butyl,
iso-Butyl, tert.-Butyl, 2-Methoxyethyl,
2-Ethoxyethyl, 2-Phenoxyethyl, 2-Hydroxyethyl,
2-Phenoxyethyl, Phenyl, o,m,p-Nitrophenyl,
o,m,p-Cyanphenyl oder o,m,p-Chlorphenyl.

Zur Herstellung der Verbindungen der Formel I kann man Verbindungen der Formel II mit Hydrazinen der Formel III umsetzen

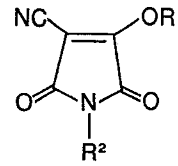

$R^1$–NH–$NH_2$

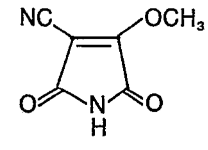

II III IV

und die entstandenen Hydrazone cyclisieren. R ist dabei der Rest eines Alkohols. Reste $R^1$ oder $R^2$ können auch ausgehend von den als Zwischenprodukten entstandenen Hydrazonen ($R^1 = H$) oder von Verbindungen der Formel I ($R^1$ oder $R^2 = H$) nachträglich eingeführt werden.

Verbindungen der Formel II sind analog der in J. Am. Chem. Soc. 80, 1385 (1958) für die Verbindung der Formel IV angegebenen Methode erhältlich.

Die Cyclisierung kann beispielsweise durch Erhitzen in Lösungsmitteln wie Methanol, Ethanol, Propanol, Butanol, Methylglykol, Ethylglykol, Butylglykol, Ameisensäure, Essigsäure oder Propionsäure durchgeführt werden, andere Säuren wie Schwefelsäure, Phosphorsäure oder Toluolsulfonsäure sind ebenfalls brauchbar.

Einzelheiten der Herstellung können den Beispielen entnommen werden, in denen sich Angaben über Teile und Prozente, sofern nicht anders vermerkt, auf das Gewicht beziehen.

Die Verbindungen der Formel I eignen sich sehr gut als Diazokomponenten zur Herstellung von Azofarbstoffen.

Beispiel 1

a) 2-Cyan-3-hydrazino-maleinimid

50 Teile 2-Cyan-3-methoxymaleinimid werden in eine Lösung von 16,5 Teilen Hydrazinhydrat in 300 Raumteilen Isopropanol unter Eiskühlung eingetragen. Anschliessend wird das Reaktionsgemisch eine halbe Stunde bei Raumtemperatur gerührt, der rote Niederschlag abgesaugt, mit Isopropanol gewaschen und getrocknet. Man erhält 47 Teile (94% der Theorie) 2-Cyan-3-hydrazinomaleinimid Schmp. $>350\,°C$, IR (KBr): 3120, 2200, 1670 $cm^{-1}$.

b) 5-Amino-pyrazolo-(3,4)-dicarboximid

30 Teile 2-Cyan-3-hydrazinomaleinimid werden in 100 Raumteilen Eisessig sieben Stunden zum Sieden erhitzt. Man lässt abkühlen, saugt den Niederschlag ab, wäscht ihn mit wenig Eisessig nach und trocknet. Man erhält 22 Teile (73% der Theorie) 5-Amino-pyrazolo-(3,4)-dicarboximid Schmp. $>350\,°C$, IR (KBr): 3300, 3120, 1670 $cm^{-1}$.

Beispiel 2

5-Amino-pyrazolo-(3,4)-N-methyldicarboximid

16,6 Teile 2-Cyan-3-methoxy-N-methylmaleinimid werden in eine Lösung von 5 Teilen Hydrazinhydrat in 80 Raumteilen n-Butanol unter Eiskühlung eingetragen. Anschliessend erhitzt man die Mischung vier Stunden zum Sieden und lässt abkühlen. Der entstandene Niederschlag wird abgesaugt, mit n-Butanol gewaschen und getrocknet.

Man erhält 15 Teile (94% der Theorie) 5-Amino-pyrazolo-(3,4)-N-methyldicarboximid. Schmp. 285–286 °C, IR (KBr): 3400, 3200, 1720, 1670 $cm^{-1}$.

Die Herstellung des als Ausgangsmaterials benötigten 2-Cyan-3-methoxy-N-methylmaleinimids wird wie folgt vorgenommen:

19 Teile des Kaliumsalzes von 2-Cyan-3-hydroxy-N-methylmaleinimid (Herstellung analog zu den in J. Am. Chem. Soc. 80, 3924 (1958) beschriebenen Salzen) und 15,3 Teile Phosphoroxidtrichlorid werden in 100 Raumteilen Schwerbenzin eine halbe Stunde zum Sieden erhitzt. Anschliessend filtriert man die heisse Mischung ab und lässt das Filtrat abkühlen. Der entstandene Niederschlag wird abgesaugt, mit Benzin gewaschen und getrocknet. Man erhält 9,6 Teile (56% der Theorie) 2-Cyan-3-chlor-N-methylmaleinimid. Schmp. 141–143 °C, IR (KBr): 2270, 1720, 1630 $cm^{-1}$.

43 Teile 2-Cyan-3-chlor-N-methylmaleinimid werden in 100 Raumteilen Methanol eingetragen und die Mischung fünfzehn Minuten zum Sieden erhitzt. Nach Abkühlen saugt man den Niederschlag ab, wäscht ihn mit wenig Methanol und trocknet. Man erhält 37 Teile (88% der Theorie) 2-Cyan-3-methoxy-N-methylmaleinimid. Schmp. 123–125 °C, IR (KBr): 2220, 1720, 1640 $cm^{-1}$.

Beispiel 3

5-Amino-1-phenyl-pyrazolo-(3,4)-dicarboximid

50 Teile 2-Cyan-3-methoxymaleinimid werden in eine Lösung von 35,6 Teilen Phenylhydrazin in 200 Raumteilen Eisessig unter Eiskühlung eingetragen. Man erhitzt anschliessend zwölf Stunden zum Sieden, lässt abkühlen, saugt den Niederschlag ab, wäscht ihn mit wenig Eisessig nach und trocknet. Man erhält 61 Teile (81% der Theorie) 5-Amino-1-phenyl-pyrazolo-(3,4)-dicarboximid. Schmp. 292–294 °C, IR (KBr): 3440, 3220, 1670 $cm^{-1}$.

Beispiel 4

5-Amino-1-phenyl-pyrazolo-(3,4)-N-methyldicarboximid

22,8 Teile 5-Amino-1-phenyl-pyrazolo-(3,4)-dicarboximid werden in 100 Raumteilen Dimethylformamid gelöst, anschliessend 6 Teile einer 30%igen Lösung von Natriummethylat in Methanol zugegeben und das Reaktionsgemisch eine halbe Stunde bei Raumtemperatur gerührt. Man gibt dann 13,8 Teile Dimethylsulfat zu, lässt noch vier Stunden bei Raumtemperatur rühren und fällt die Mischung mit 400 Teilen Wasser. Das Produkt wird abgesaugt, mit Wasser gewaschen und getrocknet. Man erhält 19,5 Teile (81% der Theorie) 5-Amino-1-phenyl-pyrazolo-(3,4)-N-methyldicarboximid. Schmp. 237–239 °C, IR (KBr): 3400, 3200, 1720, 1640 $cm^{-1}$.

Beispiel 5

5-Amino-1-methyl-pyrazolo-(3,4)-N-methyldicarboximid

Wie in Beispiel 2 beschrieben aus 16,6 Teilen 2-Cyan-3-methoxy-N-methylmaleinimid, 4,6 Teilen Methylhydrazin und 80 Raumteilen n-Butanol.

Ausbeute: 13,2 Teile (73% der Theorie).

Schmp. 240–242 °C, IR (KBr): 3400, 3350, 3250, 1750, 1670, 1620 $cm^{-1}$.

Beispiel 6

5-Amino-1-acetyl-pyrazolo-(3,4)-N-methyldicarboximid

Wie in Beispiel 1 beschrieben, erhält man aus 8,3 Teilen 2-Cyan-3-methoxy-N-methylmaleinimid und 3,7 Teilen Acethydrazid in 50 Raumteilen Isopropanol 8,7 Teile (84% der Theorie) 2-Cyan-3-(2'-acethydrazino)-N-methylmaleinimid. Schmp. 220–223 °C, IR (KBr): 3220, 2250, 1720, 1660 $cm^{-1}$. Durch siebenstündiges Erhitzen zum Sieden in 40 ml Propionsäure wird dieses zum 5-Amino-1-acetyl-pyrazolo-(3,4)-N-methyldicarboximid cyclisiert.

Ausbeute: 6,8 Teile (78% der Theorie)

Zersetzung bei 300 °C, IR (KBr): 3250, 1720, 1650 $cm^{-1}$.

## Patentansprüche

1. Aminopyrazolverbindungen der allgemeinen Formel I

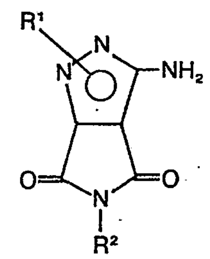

I

in der

$R^1$ Wasserstoff, $C_1$- bis $C_4$-Alkyl, Cyanethyl, Cyclohexyl, $C_1$- bis $C_4$-Alkanoyl, gegebenenfalls durch Chlor, Cyan oder Nitro substituiertes Benzoyl, $C_1$- bis $C_4$-Alkoxycarbonyl, Phenoxycarbonyl, Carbamoyl, Sulfamoyl, durch $C_1$- bis $C_4$-Alkyl oder Phenyl N-mono- oder N,N-disubstituiertes Carbamoyl oder Sulfamoyl, Thiocarbamoyl $C_1$- bis $C_4$-Alkylsulfonyl, Phenyl- oder Tolylsulfonyl, gegebenenfalls durch Chlor, Cyan, Nitro, Methyl, Methoxy, Ethoxy, $C_1$- bis $C_4$-Alkanoyl, Benzoyl, $C_1$- bis $C_4$-Alkoxycarbonyl, Phenoxycarbonyl, Carboxyl, $C_1$- bis $C_4$-Alkyloxysulfonyl, Phenoxysulfonyl, Carbamoyl, Sulfamoyl oder durch $C_1$- bis $C_4$-Alkyl oder Phenyl N-mono- oder N,N-disubstituiertes Carbamoyl oder Sulfamoyl substituiertes Phenyl oder Naphthyl und

$R^2$ Wasserstoff, $C_1$- bis $C_8$-Alkyl, Cyclohexyl, Allyl, $C_1$- bis $C_4$-Alkoxy-$C_2$- oder $C_3$-alkyl, Hydroxy-$C_2$- oder $C_3$-alkyl, Phenoxy-$C_2$- oder $C_3$-alkyl, Benzyl, Phenylethyl oder gegebenenfalls durch Chlor, Cyan oder Nitro substituiertes Phenyl sind.

2. Verwendung der Verbindung gemäss Anspruch 1 als Diazokomponenten.

## Claims

1. An aminopyrazole compound of the general formula I

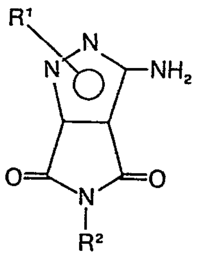

I

where

$R^1$ is hydrogen, $C_1$–$C_4$-alkyl, cyanoethyl, cyclohexyl or $C_1$–$C_4$-alkanoyl, or is benzoyl which is unsubstituted or substituted by chlorine, cyano or nitro, or is $C_1$–$C_4$-alkoxycarbonyl or phenoxycarbonyl, or sulfamyl or carbamyl which is unsubstituted or N-monosubstituted or N,N-disubstituted by $C_1$–$C_4$-alkyl or phenyl, or is thiocarbamyl, $C_1$–$C_4$-alkylsulfonyl, phenylsulfonyl or tolylsulfonyl, or is naphthyl or phenyl which is unsubstituted or substituted by chlorine, cyano, nitro, methyl, methoxy, ethoxy, $C_1$–$C_4$-alkanoyl, benzoyl, $C_1$–$C_4$-alkoxycarbonyl, phenoxycarbonyl, carboxyl, $C_1$–$C_4$-alkoxysulfonyl or phenoxysulfonyl or by sulfamyl or carbamyl which is unsubstituted or N-monosubstituted or N,N-disubstituted by $C_1$–$C_4$-alkyl or phenyl, and

$R^2$ is hydrogen, $C_1$–$C_8$-alkyl, cyclohexyl, allyl, $C_1$–$C_4$-alkoxy-$C_2$–$C_3$-alkyl, hydroxy-$C_2$–$C_3$-alkyl, phenoxy-$C_2$–$C_3$-alkyl, benzyl or phenylethyl, or is phenyl which is unsubstituted or substituted by chlorine, cyano or nitro.

2. The use of a compound as claimed in claim 1 as a diazo component.

## Revendications

1. Composés d'aminopyrazole répondant à la formule générale I

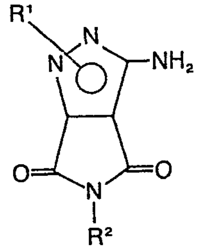

I

dans laquelle

$R^1$ est de l'hydrogène, un groupement alkyle en $C_1$–$C_4$, cyanéthyle, cyclohexyle, . alcanoyle en $C_1$–$C_4$, benzoyle éventuellement substitué par du chlore, ou un radical cyano ou nitro; alcoxycarbonyle en $C_1$–$C_4$, phénoxycarbonyle, carbamoyle, sulfamoyle; carbamoyle ou sulfamoyle N-mono- ou N,N-disubstitués par un groupement phényle ou alkyle en $C_1$–$C_4$; thiocarbamoyle, alkylsulfonyle en $C_1$–$C_4$, phénylsulfonyle ou tolylsulfonyle; phényle ou naphtyle éventuellement substitués par un atome de chlore ou un groupement cyano, nitro, méthyle, méthoxy, éthoxy, alcanoyloxy en $C_1$–$C_4$, benzoyle, alcoxycarbonyle en $C_1$–$C_4$, phénoxysulfonyle, carbamoyle, sulfamoyle ou carbamoyle ou sulfamoyle N-mono- ou N-disubstitués par un radical alkyle en $C_1$–$C_4$ ou phényle,

$R^2$ est de l'hydrogène ou un groupement alkyle en $C_1$–$C_8$, cyclohexyle, allyle, (alcoxy en $C_1$–$C_4$)-alkyle en $C_2$ ou $C_3$, hydroxyalkyle en $C_2$ ou $C_3$, phénoxyl-(alkyle en $C_2$ ou $C_3$), benzyle, phényléthyle ou phényle éventuellement substitué par du chlore ou un radical cyano ou nitro.

2. Utilisation des composés selon la revendication 1, comme composants diazoïques.